# EUROPÄISCHE PATENTSCHRIFT

(11) **EP 0 665 745 B1**
(45) Veröffentlichungstag und Bekanntmachung des Hinweises auf die Patenterteilung: **15.04.1998**
(21) Anmeldenummer: 94900072.3
(22) Anmeldetag: 20.10.1993
(51) Int. Cl.: A61K 9/70

(54) **PFLASTER ZUR TRANSDERMALEN VERABREICHUNG VON FLÜCHTIGEN, PHARMAZEUTISCH WIRKSAMEN, CHEMISCH BASISCHEN INHALTSSTOFFEN UND VERFAHREN ZU SEINER HERSTELLUNG**
PLASTER FOR THE TRANSDERMAL ADMINISTRATION OF VOLATILE, PHARMACEUTICALLY ACTIVE, CHEMICALLY ALKALINE INGREDIENTS, AND PROCESSS FOR PRODUCING THE SAME
EMPLATRE POUR ADMINISTRATION PERCUTANEE D'INGREDIENTS VOLATILS, CHIMIQUEMENT BASIQUES, PHARMACEUTIQUEMENT ACTIFS, ET SON PROCEDE DE PREPARATION

(30) Priorität: 22.10.1992 EP 92118036
(43) Veröffentlichungstag der Anmeldung: 09.08.1995
(73) Patentinhaber: LTS LOHMANN Therapie-Systeme GmbH, 56567 Neuwied (DE)
(72) Erfinder: WOLTER, Karin, D-56581 Melsbach (DE); MÜLLER, Walter, D-56564 Neuwied (DE); SIMON, Günter, D-54576 Hillesheim (DE); NALBACH, Christa, D-56599 Leutesdorf (DE); HOFFMANN, Rainer, D-56566 Neuwied (DE)
(74) Vertreter: Flaccus, Rolf-Dieter, Dr.
(86) Internationale Anmeldenummer: EP9302899
(87) Internationale Veröffentlichungsnummer: WO9408571

(56) Entgegenhaltungen:
- WO-A-89/09051
- US-A- 3 797 494

## Beschreibung

Die Erfindung betrifft ein neues Pflaster zur transdermalen Verabreichung von flüchtigen, pharmazeutisch wirksamen, chemisch basischen Inhaltsstoffen sowie ein Verfahren zu seiner Herstellung.

Eine Reihe von sehr wertvollen pharmazeutisch wirksamen Substanzen ist chemisch basischer Natur. Von diesen Stoffen ist eine erhebliche Anzahl unter Umgebungsbedingungen flüchtig, so z.B. Deprenyl, Tolbuterol, Propanolol, Bupranolol, Arecolin, Verapamil, Methamphetamin und Amphetaminil, und insbesondere Deprenyl (Selegilin), einer in der Behandlung der Parkinsonschen Krankheit wohlbekannte Verbindung.

Es ist bekannt, Deprenyl oder eines seiner Salze oral zu verabreichen, es wurde aber auch vorgeschlagen, diese transdermal zu verabreichen (US-Patente 4,861,800 und 4,868,218 sowie deren europäisches Äquivalent EP 0 406 488 und die Internationale Anmeldung WO 89/09051. Darüber hinaus ist aus dem US-Patent 4,812,481 (EP 0 241 809) bekannt, Selegilin zusammen mit Amantadin zu verabreichen, eine Kombination, die eine synergistische Wirkung haben soll, und zwar in Medikamenten, für die orale, parenterale, rektale und vaginale Verabreichung oder die Verabreichung an die Haut und Schleimhäute (z.B. als Lösungen, Lotionen, Emulsionen, Salben (Unguenta), Pflaster etc. ).

Das Dokument US-A-3,797,494 offenbart Pflaster für die transdermale Verabreichung von pharmazeutisch wirksamen Substanzen. Es handelt sich dabei um nicht-flüchtige Wirkstoffe, deren Freisetzung durch Kontrollmittel über einen längeren Zeitraum der Applikation eingestellt wird. Es handelt sich dabei um mikroporöses Material, beispielsweise für eine Kontrollmembran oder für wirkstoffhaltige Mikro-Kapseln. Dabei richtet sich die Art des mikroporösen Materials nach den besonderen Eigenschaften des Wirkstoffs und die Poren können eine effektive Porengröße zwischen 10 2,34 Ångströms bis 100 Mikrons haben.

Untersuchungen der Anmelderin haben ergeben, daß die in der internationalen Anmeldung WO 89/09051 beschriebene Verfahrensweise jedoch völlig unzufriedenstellend und sogar mangelhaft ist. Entsprechend dem Beispiel dieser Veröffentlichung, werden die Bestandteile in ein Lösungsmittel, wie z.B. Aceton oder Ethanol, gegeben und zu einer viskosen Masse vermischt. Diese Masse wird dann unter Verwendung einer herkömmlichen Vorrichtung auf eine aluminisierte Polyesterfolie (Dicke 23 Mikron) gestrichen, so daß eine Folie von - im nassen Zustand - 0,2 mm Dicke entsteht. Die Folie wird dann 4-6 Stunden lang bei Raumtemperatur getrocknet.

Die Untersuchungen der Anmelderin haben ergeben, daß in dem so hergestellten Produkt ein wesentlicher Teil des Wirkstoffes durch Verdunstung verlorengeht, so daß es schließlich wesentlich weniger Wirkstoff enthält als zu Beginn eingesetzt wurde, außerdem unterschieden sich die Produkte von Wiederholversuchen wesentlich voneinander. Darüberhinaus ist es für den Fachmann offensichtlich, daß aus technischer und kommerzieller Sicht eine Trocknung von 4 bis 6 Stunden entsprechend WO 89/09051 völlig nachteilig ist.

Es ist Ziel der Erfindung, die Nachteile des Standes der Technik zu überwinden und ein Pflaster von standardisierter Qualität für die transdermale Verabreichung von Deprenyl zur Verfügung zu stellen.

Allgemein ausgedrückt betrifft die Erfindung ein Pflaster zur transdermalen Verabreichung eines flüchtigen, pharmazeutisch wirksamen, chemisch basischen Inhaltsstoffes, vorzugsweise Deprenyl, welches ein Mehrfachelement-System enthält, umfassend
(a) eine Matrix, in der als Wirkstoff physiologisch akzeptable Salze des flüchtigen Wirkstoffs verteilt sind, wobei die Matrix einen Haftkleber enthält,
(b) eine haftklebende Zusammensetzung, die basische Gruppen zur Freisetzung der freien Base aus ihrem Salz enthält,
(c) eine für die diffundierbaren Inhaltsstoffe von (a) und (b) undurchlässige Rückschicht und
(d) eine für die diffundierbaren Inhaltsstoffe von (a) und (b) undurchlässige abziehbare Schutzfolie,
wobei die Matrix (a) oder zumindest ein Teil von (b), je nachdem, ob die Matrix (a) oder der Teil von (b) Kontakt mit der Abziehfolie (d) hat, eine Klebrigkeit (Tack) aufweist, die für die Befestigung des Pflasters auf der Haut ausreicht, und wobei jeder Teil von (b) zwischen Matrix (a) und Abziehfolie (d) für den Wirkstoff, vorzugsweise Deprenyl oder einer seiner Salze oder für beide, durchlässig ist. Zweckmäßigerweise sind die Matrix (a) und die Teile von (b) zusammenlaminiert, wobei zum Zeitpunkt der Laminierung das Salz nur in (a) enthalten ist.
Im Herstellungsprozess des Pflasters nach der Erfindung wird Element (a) bis zur Verarbeitung mit Element (b) unter solchen Bedingungen gehalten, daß sich das Salz nicht zersetzt, wobei jede Schicht von (b) zwischen Element (a) und Abziehfolie (d) für den Wirkstoff durchlässig ist.

In einer bestimmten Ausführungsform betrifft die Erfindung außerdem ein Verfahren zur Herstellung eines Pflasters entsprechend Anspruch 1, umfassend
(i) das Herstellen einer Mischung aus einem physiologisch unbedenklichen Salz eines flüchtigen, pharmazeutisch wirksamen Inhaltsstoffes und einer flüssigen Zusammensetzung aus einem Haftklebermaterial mit einem Lösungs- oder Verdünnungsmittel, und Verdampfen des Lösungs- oder Verdünnungsmittels aus dieser Mischung, so daß eine Matrix (a) aus einem homogenen Substrat in Schicht- oder partikulärer Form entsteht,
(ii) Herstellen einer flüssigen Zusammensetzung, enthaltend ein Lösungs- oder Verdünnungsmittel und die genannte haftklebende Zusammensetzung, die basische Gruppen enthalten kann, welche geeignet sind, die freie Base aus ihrem Salz zu bilden, Aufschichten der Kleberzusammensetzung zu wenigstens einer Schicht, die einen Lochbereich von definierter geometrischer Form aufweisen kann, und Verdampfen des Lösemittels oder Verdünnungsmittels aus dieser Schicht, so daß eine Schicht (b) entsteht,
(iii) Laminieren der Matrix (a) mit Schicht (b),
(iv) Auftragen der oben genannten Rückschicht (c) und der oben genannten Abziehfolie (d) auf die Matrix (a) und Schicht (b), so, daß entweder die Matrix (a) oder eine für die freie Base durchlässige Schicht von (b) direkten Kontakt mit der Abziehfolie (d) hat,
wobei die mit dem Wirkstoff beladene Matrix (a) und wenigstens eine Schicht (b) eine Klebrigkeit aufweisen, die zur Befestigung des Pflasters auf der Haut ausreicht, wobei die Matrix (a) bis zur Verarbeitung mit der Zusammensetzung (b) unter solchen Bedingungen gehalten wird, daß das oben genannte Salz nicht zersetzt wird, und wobei die Verfahrensschritte (i) und (ii) in beliebiger Reihenfolge durchgeführt werden. Ein weiteres Ziel der Erfindung besteht in einem Verfahren zur Behandlung eines an der Parkinsonschen oder Alzheimerschen Krankheit leidenden Patienten, welches die Behandlung des Patienten mit einem Pflaster wie oben definiert umfaßt.

Geeignete Wirkstoffe zur Verarbeitung in den erfindungsgemäßen Pflastern sind z.B. Tolbuterol, Propanolol, Arecolin, Verapamil, Methamphetamin, Amphetaminil, Clenbuterol, Gallopamil und vorzugsweise Deprenyl sowie andere, dem Fachmann bekannte Substanzen.

Die Matrix (a) kann anfangs oder nach der Lagerung auch die freie Base anstelle des oder zusätzlich zu dem Salz enthalten, jedoch ist es bevorzugt, daß anfangs wenigstens ein Teil des Wirkstoffes, vorzugsweise die ganze Menge des Wirkstoffes, in Form eines physiologisch akzeptablen Salzes vorliegt, z.B. Hydrogenfumarat, Hydrogenmalonat oder Hydrogensuccinat, oder als ein Salz einer Mineralsäure wie Sulfat, Phosphat, jedoch insbesondere ein Halogenwasserstoffsalz und vor allem insbesondere das Hydrochlorid oder Hydrobromid. Wird ein Salz verwendet, so kann seine Diffusionsfähigkeit durch die begleitende Verwendung eines herkömmlichen Lösungsvermittlers, wie z.B. Glycerin, 1,2-Propandiol, Monomethyl- oder Monoethylether von Diethylenglykol, 2-Octyldodecanol, Sorbitol-Laurat, -Palmitat, -Stearat oder -Oleat, C₈/C₁₀-ethoxylierte Glyceride und ethoxylierte Oleinglyceride erhöht werden.

Der Wirkstoff kann in seiner freien (Basen-)Form in Element (b) alleine oder zusätzlich zur Matrix (a) enthalten sein. Element (b) kann aus einem Amino-haltigen polymerisierbaren Monomer oder einer Mischung aus einem adhäsiven Polymer und einer Base oder einer Kombination daraus gebildet werden. Eine geeignete Base ist ein nichtflüchtiges Amin, wie z.B. Aminoalkohole, z.B. Mono- und Diethylamin, insbesondere jedoch ein Amino-enthaltendes Polymer. Die basischen Gruppen von (b) dienen schließlich zur Freisetzung der freien Base aus der Schicht (a).

Element (b) kann eine einzige Schicht oder mehrere, insbesondere 2 bis 3, Schichten umfassen, von denen eine oder mehrere basisch sind. Element (b) kann ebenfalls kreisrund geformt sein und die Matrix (a) umgeben und diese möglicherweise auch zur Seite der Rückschicht hin abdecken. Anders ausgedrückt kann die Matrix (a) nach allen außer einer Seite hin von Element (b) umgeben sein, wobei die verbleibende Seite für das Anbringen auf der Haut mit Hilfe der Klebaktivität von Element (a) vorgesehen ist. Mit Vorteil ist die Matrix (a) jedoch zwischen die beiden Schichten von (b) laminiert. In einer bevorzugten Ausführungsform ist jedoch eine Schicht (b) auf einer Seite der Matrix (a), und eine andere Schicht (b) ist auf der anderen Seite der Matrix (a). In diesem Fall weist die Schicht (b), die sich näher an der Abziehfolie (d) befindet, häufig eine größere Klebrigkeit und einen geringeren Amingehalt auf als die Schicht (b), die von der Abziehfolie (d) weiter entfernt ist. In einer bevorzugten Ausführungsform ist Element (b) in direktem Kontakt mit der Abziehfolie (d).

Ist der Wirkstoff Deprenyl, so hat dieser mit Vorteil L-Form. Er liegt in der Regel in einer Menge von etwa 5 bis 75, vorzugsweise etwa 10 bis 50 mg pro Pflaster vor, berechnet als freie Base, wobei die Größe des Pflasters in einem Bereich von etwa 10 bis 25, vorzugsweise etwa 12 bis 20 cm² liegt. Die Mengen der übrigen Wirkstoffe sind abhängig von ihrer wirksamen Dosis, sie können jedoch auch innerhalb weiter Grenzen variieren, z.B. in den oben angegebenen Grenzen.

Das Element (b) kann zusätzlich Inhaltsstoffe zur Verbesserung der Permeation, Verteilung und/oder Penetration der Wirkstoffe enthalten. Geeignete Permeationsverbesserer sind die oben als Lösungsmittel genannten Stoffe, darüberhinaus können aber auch andere herkömmliche Enhancer verwendet werden, wie Ester der Formel [CH₃(CH₂)ₘCOO]ₙR, in der m eine ganze Zahl von 8 bis 16, vorzugsweise von 8 bis 12; n 1 oder 2, vorzugsweise 1; und R ein niedriger Alkyl(C₁-C₃)rest ist, der durch bis zu 2 Hydroxylgruppen substituiert sein kann, oder ein Gemisch aus einem solchen Ester oder Methyllaurat und Diethylenglykolmonomethyl- oder -monoethylether. Bevorzugte Ester sind niedrige Alkyl(C₁-C₃)ester der Laurinsäure, wie Propylenglykolmonolaurat (PGML). Andere geeignete Enhancer sind Laurinsäure, Caprinsäure, Ölsäure, Glycerinoleat, höhere Alkohole, wie Dodecanol, Undecanol, Octanol, Ester von Carbonsäuren, in denen der alkoholische Bestandteil ein polyethoxylierter Alkohol sein kann, Diester von Dicarbonsäuren, wie Di-n-butyladipat und Triglyceride, insbesondere Triglyceride der Capryl/Caprinsäure mit mittlerer Kettenlänge , oder Kokosöl, mehrwertige Alkohole, z.B. Glycerol und 1,2-Propandiol, die mit Polyethylenglykolen verethert sind.

Elemente (a) und/oder (b) können des weiteren zusätzliche Wirkstoffe enthalten, z.B. Amantadin zusammen mit Deprenyl, und/oder andere Substanzen wie z.B. Farbstoffe, Geschmacksstoffe und Konservierungsmittel, Füllstoffe etc. enthalten.

Die Anordnung der Elemente (a) und (b) kann in großem Maße variieren. In einer bevorzugten Ausführungsform hat Element (b) direkten Kontakt mit der Rückschicht (c). Die Schicht (c) und/oder die Abziehfolie (d) können eine Polyesterfolie enthalten, die aluminisiert sein kann, oder eine aluminisierte Folie eines synthetischen Harzes, wie Polypropylen, Nylon, Polycaprolactam oder Silikonfolie.

Das Gewicht des gesamten Pflasters kann zwischen ca. 0,05 und 10, vorzugsweise ca. 0,1 und 5 g variieren, und die Dicke zwischen ca. 0,1 und 10, vorzugsweise ca. 0,5 bis 5 mm.

Im folgenden wird die Erfindung anhand der begleitenden Zeichnungen beschrieben, in denen
- Fig. 1: einen Querschnitt durch eine Ausführungsform eines erfindungsgemäßen Pflasters darstellt,
- Fig. 2: einen Querschnitt durch eine andere Ausführungsform des erfindungsgemäßen Pflasters darstellt;
- Fig. 3: einen Querschnitt durch eine weitere Ausführung des erfindungsgemäßen Pflasters darstellt, in der Element (b) an die Rückschicht angrenzend angeordnet ist und die Matrix (a) zwischen Element (b) und der Abziehfolie angeordnet ist;
- Fig. 4: einen Querschnitt durch eine weitere Ausführungsform des erfindungsgemäßen Pflasters darstellt, in der die Positionen des Elementes (b) und der Matrix (a) im Vergleich zu Fig. 3 miteinander vertauscht sind;
- Fig. 5: einen Querschnitt durch eine weitere Ausführungsform des erfindungsgemäßen Pflasters darstellt, in der Element (b) die Matrix (a) umgibt und diese zur Seite der Rückschicht hin abdeckt;
- Fig. 6: eine Draufsicht des Pflasters entlang der Linie zwischen X und Y in Fig. 5 zeigt;
- Fig. 7 und Fig. 8: Ausführungsformen wie diejenigen in Fig. 5 und 6 zeigen, jedoch mit vertauschten Positionen der Matrix (a) und des Elementes (b);
- Fig. 9: verschiedene Draufsichten des Matrixteils von Fig. 5 entlang der Linie zwischen X und Y bzw. dem Element(b)teil der Fig. 5 zeigt;

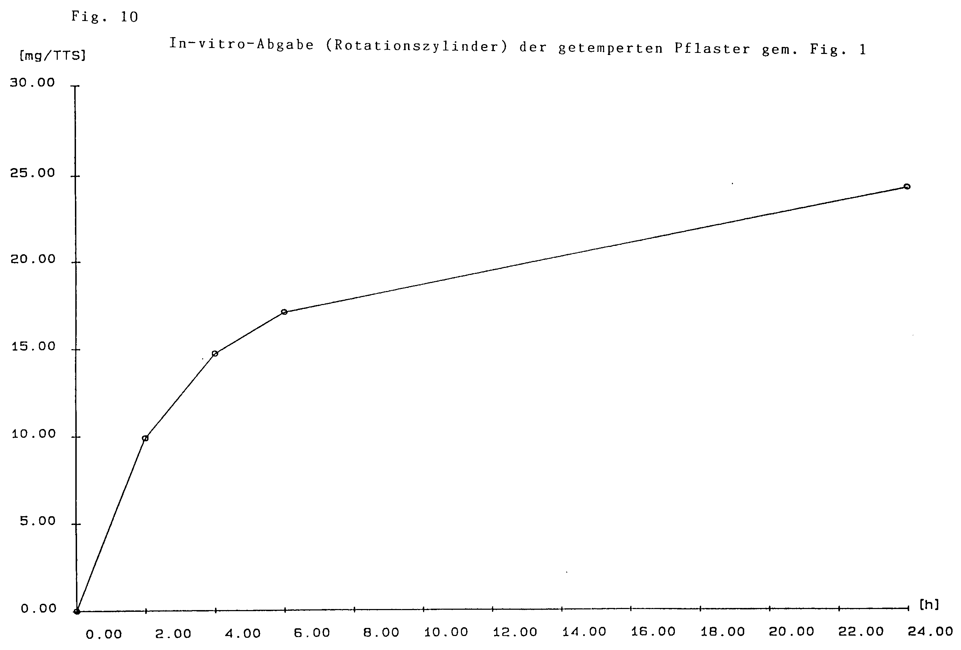

- Fig. 10: eine Kurve der In-vitro-Abgabe entsprechend dem bekannten Rotationszylinder-Verfahren der "getemperten" Pflaster gemäß Fig. 1 darstellt;

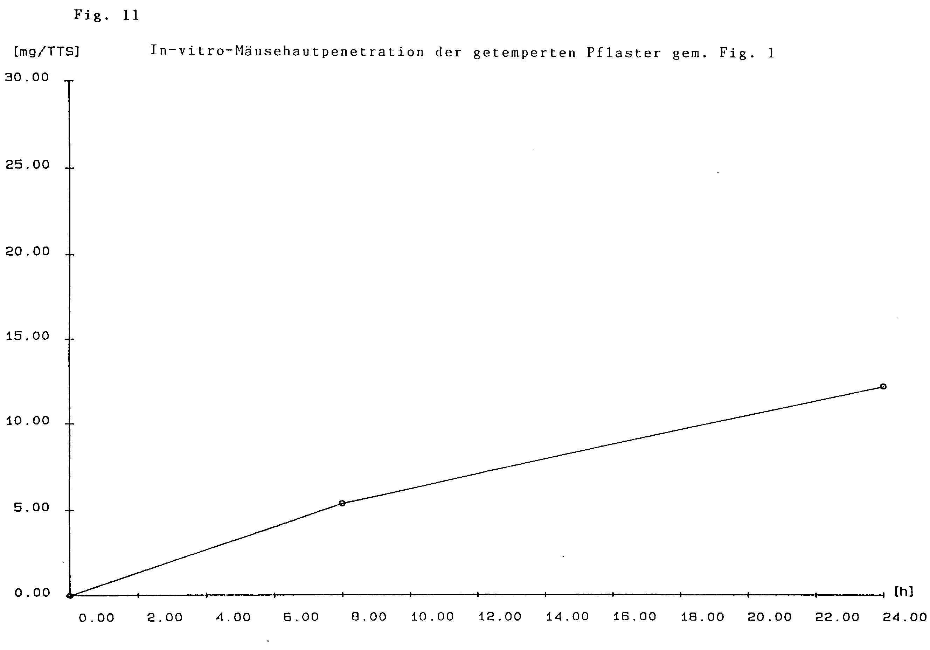

- Fig. 11: eine Kurve der In-vitro-Mäusehautpenetration der getemperten Pflaster gemäß Fig. 1 zeigen. Die Kurven der Fig. 10 und 11 geben beide die Durchschnittsresultate von drei Versuchen wieder.

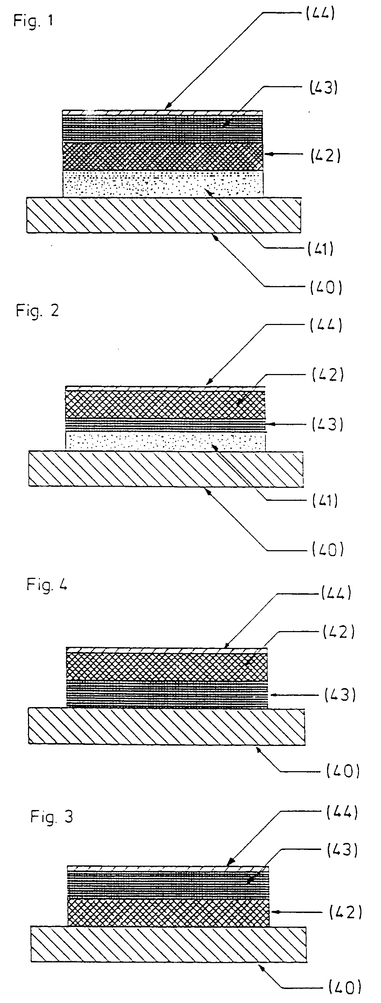

Im folgenden werden die Zeichnungen eingehender erläutert: In Fig. 1 ist ein erfindungsgemäßes Pflaster dargestellt, umfassend eine wiederablösbare Abziehfolie 40, an welcher die Schicht 41 des Elementes (b) haftet, die anfänglich frei von Wirkstoff ist. An Schicht 41 haftet eine Schicht 42 der Matrix (a), die den Wirkstoff in Salzform enthält. Diese wiederum trägt eine adhäsive Schicht 43 des Elementes (b), die an einer undurchlässigen Rückschicht (c) 44 anhaftet.

In der Variante gemäß Fig. 2, sind die Schichten 42 der Matrix (a) und 43 des Elements (b) miteinander vertauscht, jedoch ist es notwendig, daß 42 an der Rückschicht 44 haftet, entweder durch das ihr inhärente Klebvermögen oder einen hinzugefügten Kleber (nicht dargestellt).

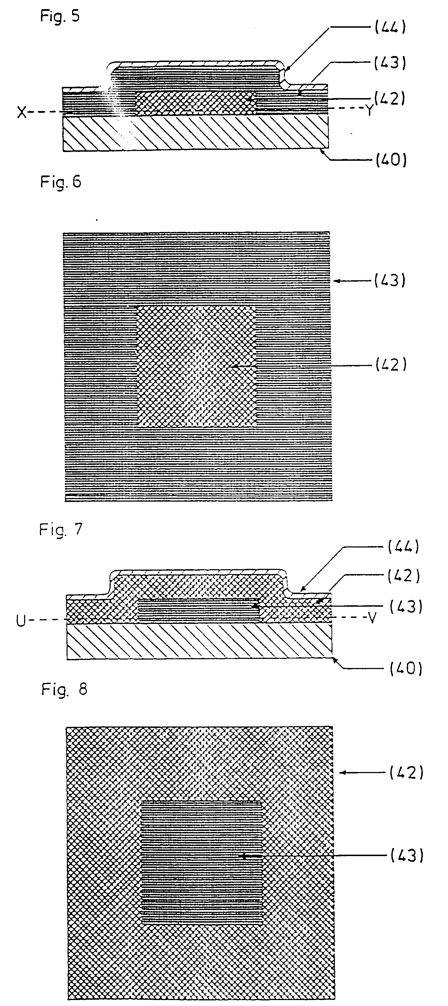

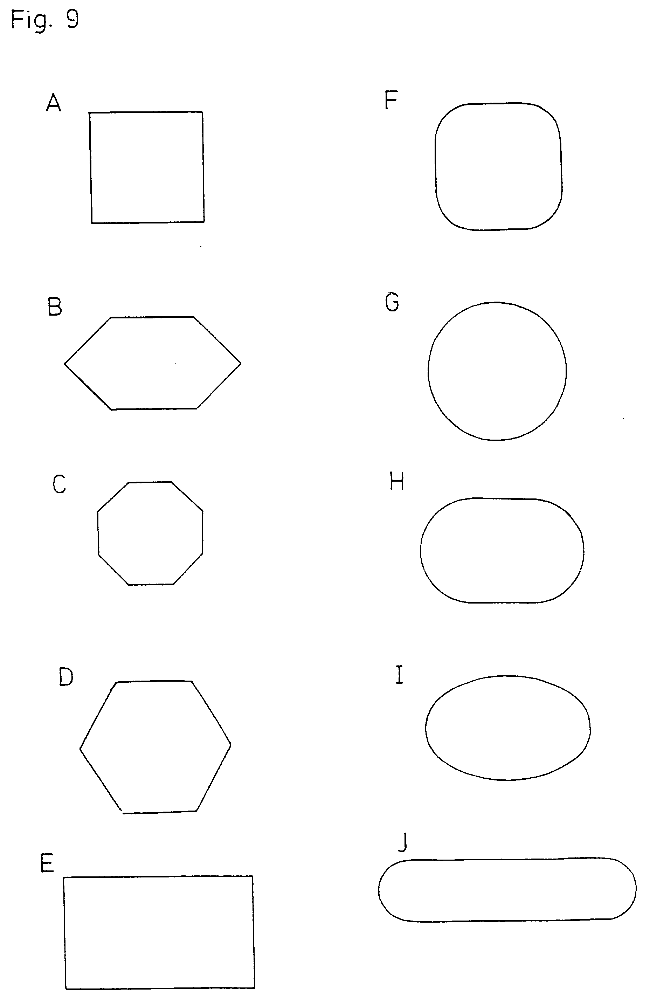

In der Variante gemäß Fig. 5 bis 9, deckt Element (b) die Matrix (a) zu der Seite der Rückschicht hin ab, jedoch könnte die Matrix (a) auch direkten Kontakt mit der Rückschicht haben. Es ist ein inhärentes Merkmal dieser Ausführung, daß ein Teil von Element (b), der für eine zufriedenstellende Adhäsion zur Haut ausreichend ist, die Matrix (a) umgibt. Matrix (a) kann wie in Fig. 6 dargestellt geformt sein oder entsprechend einer der Figuren 9B-J (die Form gemäß Fig. 9A ist identisch mit der in Fig. 6).

Die Erfindung wird anhand der folgenden, erläuternden Beispiele näher beschrieben.

### BEISPIEL 1

1.1 Ein auf Lösungsmittel basierender, nichtvernetzender Haftkleber auf Acrylatbasis, löslich in Ethylacetat (Handelsname DURO-TAK 280-2287) und mit einem Feststoffgehalt von 50-52 % und einer Viskosität von 10.000-24.000 mPa·s bei 25 °C wird in einer Konzentration von 50 Gew.-% in Ethylacetat gelöst. 800 g dieser Lösung werden mit 2000 g Deprenylhydrochlorid und 3700 g Ethylacetat vereinigt und eine Dispersion hergestellt. Zusätzliche ca. 7200 g der Acrylatpolymerlösung werden der Dispersion unter Rühren und Erhitzen hinzugefügt, und dann 1400 g Ethylacetat verdampft.
1.2 Getrennt davon werden 3200 g eines Copolymers aus Dimethylaminoethylmethacrylat und neutralen Methacrylsäureestern (Handelsname Eudragit E), z.B. Ester der Alkylalkohole mit 1 bis 4 Kohlenstoffatomen, wie Methyl-, Ethyl- und Isopropyl-, die verschiedenen Butyl- (n-, sek-, iso-, tert-) und Hexyl-, in 3200 g Methylethylketon gelöst. Danach werden 22.800 g eines selbstvernetzenden Haftklebers auf Acrylatbasis (Handelsname DURO-TAK 280-2516), mit einem Feststoffgehalt von 41-43 % in einem Lösungsmittelgemisch aus 64 % Ethylacetat, 25 % Ethanol, 9 % Heptan und 2 % Methanol, und mit einer Viskosität von 3.000 bis 7.000 Mpa·s bei 25 °C, hinzugefügt.
1.3 Mit der Lösung aus (1.2) wird eine erste wiederabziehbare Folie beschichtet und das Lösungsmittel verdampft bis 64 g/m² Feststoffe zurückbleiben. Die beschichtete Seite der ersten Folie wird auf einer 15 µ dicken Polyester-Rückschicht (c) aufgebracht.
1.4 Getrennt davon wird die Dispersion aus 1.1 auf eine zweite wiederabziehbare Folie in einer Menge, die 62 g/m² nicht-flüchtiges Material enthält, schichtförmig aufgetragen. Das Lösungsmittel wird verdampft. Die Beschichtung der zweiten Folie wird nach dem Entfernen der ersten Folie von dem Produkt aus 1.3 auf die beschichtete Seite der Polyester-Rückschicht (c) auflaminiert. Das Zwischenprodukt umfaßt in dieser Phase eine Polyester-Rückschicht, eine Deprenyl-freie basische Polymerschicht, eine Deprenylenthaltende Polymerschicht und die zeitweilige zweite Folie.
1.5 Die Lösung aus (1.2) wird schichtförmig auf eine Abziehfolie (200 Mikron dicke Polyesterschicht) aufgetragen und ergibt 64 g/m² nichtflüchtiges Material. Nach dem Verdampfen der flüchtigen Stoffe wird auf das verbleibende Material eine dritte wiederabziehbare Folie plaziert.
1.6 Die temporäre zweite Folie aus (1.4) und eine dritte Folie aus (1.5) werden entfernt und das verbleibende Material laminiert. Das erhaltene Produkt ist in Fig. 1 dargestellt.

Das Produkt wird in einzelne Pflaster von 41 x 41 mm geschnitten und verpackt. Die Pflaster sind nach einer Lagerung von einigen Wochen zur Verabreichung geeignet; die Dauer der Lagerung ist von der angewendeten Lagerungstemperatur abhängig. Derartige Produkte sind in hohem Maße uniform und enthalten im wesentlichen die gesamte, anfänglich eingesetzte Wirkstoffmenge.

In im allgemeinen ähnlicher Weise können die Produkte der Fig. 2, 3 und 4 hergestellt werden.

Die in Fig. 3 und 4 gezeigten Produkte können wie folgt hergestellt werden: Die beiden Schichten 42 und 43 werden in ähnlicher Weise hergestellt wie die in Fig. 1 und 2, und das System wird dann wie mit Bezug auf Fig. 1 beschrieben weiterbehandelt.

Die in Fig. 5 und 7 dargestellten Produkte können ähnlich wie mit Bezug auf Fig. 3 und 4 beschrieben hergestellt werden. Jedoch werden die beiden Schichten so kombiniert, daß in der Ausführungsform von Fig. 5 die Wirkstoff-beladene Matrix und in der Ausführungsform gemäß Fig. 7 das haftklebende Element (b) in einer der geometrischen Formen von Fig. 9A-J ausgestanzt werden, das resultierende Produkt wird dann im Falle von Fig. 5 mit einem haftklebenden Element (b) und im Falle von Fig. 7 mit einer Matrix (a) laminiert.

## Patentansprüche

1. Pflaster für die transdermale Verabreichung von flüchtigen, pharmazeutisch wirksamen, chemisch basischen Inhaltsstoffen in Form eines Mehrfachelement-Systems, umfassend
(a) eine Matrix, in der als Wirkstoff physiologisch akzeptable Salze des flüchtigen Wirkstoffs verteilt sind, wobei die Matrix einen Haftkleber enthält,
(b) eine haftklebende Zusammensetzung, die Aminogruppen enthält, zur Freisetzung der freien Base aus ihrem Salz,
(c) eine für die diffundierbaren Inhaltsstoffe von (a) und (b) undurchlässige Rückschicht und
(d) eine für die diffundierbaren Inhaltsstoffe von (a) und (b) undurchlässige Abziehfolie,
wobei die Matrix (a) oder zumindest ein Teil von (b), je nachdem, ob die Matrix (a) oder der Teil von (b) Kontakt mit der Abziehfolie (d) hat, eine Klebrigkeit (Tack) aufweist, die für die Befestigung des Pflasters auf der Haut ausreicht, und wobei jeder zwischen Matrix (a) und Abziehfolie (d) angebrachte Teil von (b) für den flüchtigen Wirkstoff oder eines seiner Salze oder für beide durchlässig ist.

2. Pflaster nach Anspruch 1, bei dem die Matrix (a) und die Teile von (b) zusammenlaminiert sind und zum Zeitpunkt der Laminierung das Salz nur in (a) und die Aminogruppen nur in den Teilen von (b) enthalten sind.

3. Pflaster entsprechend Anspruch 1 oder 2, worin (a) oder (b) oder beide den Wirkstoff zusätzlich in Form der freien Base enthalten.

4. Pflaster entsprechend einem oder mehreren der Ansprüche 1 bis 3, in dem das Salz das Hydrochlorid oder das Hydrobromid oder beide umfaßt.

5. Pflaster entsprechend einem oder mehreren der Ansprüche 1 bis 4, in dem die Matrix (a) einen Lösungsvermittler für das physiologisch akzeptable Salz oder einen Permeationsverbesserer oder beides enthält.

6. Pflaster entsprechend einem oder mehreren der Ansprüche 1 bis 5, in dem der Wirkstoff Deprenyl, vorzugsweise in L-Form, ist, wobei der Deprenyl-Bestandteil zweckmäßig in einer Menge von ca. 5 bis ca. 75 mg, berechnet als Deprenylbase, vorliegt.

7. Pflaster entsprechend einem oder mehreren der Ansprüche 1 bis 6, in dem das Element (b) eine Vielzahl von Schichten umfaßt und vorzugsweise aus 2 bis 3 Schichten besteht, von denen wenigstens eine basische Gruppen enthält, wobei die Matrix (a) zweckmäßig zwischen 2 Schichten des Elementes (b) laminiert ist, von denen mit Vorteil eine Schicht B₁ auf der Abziehfolie (d) und eine Schicht B₂ auf der Rückschicht (c) angeordnet ist, wobei Schicht B₁ eine größere Klebaktivität aufweist als Schicht B₂, und wobei diejenige Schicht (b), die sich in größerer Nähe zur Abziehfolie (d) befindet, vorzugsweise einen geringeren Gehalt an basischen Gruppen aufweist als diejenige Schicht (b), die von der Abziehfolie (d) weiter entferne ist.

8. Pflaster entsprechend einem oder mehreren der Ansprüche 1 bis 7, in dem die Matrix (a) einen Haftkleber enthält und in dem vorzugsweise Element (a) entweder an allen außer an einer Seite von Element (b) umgeben ist, wobei die verbleibende Seite für die Befestigung auf der Haut mit Hilfe der Klebaktivität des Elementes (a) vorgesehen ist, oder so von Element (b) umgeben ist, daß die Matrix (a) direkten Kontakt mit der Rückschicht (c) hat.

9. Pflaster entsprechend einem oder mehreren der Ansprüche 1 bis 8, in dem das Element (b) ein adhäsives Polymer enthält, das Aminogruppen enthält, welche die basischen Gruppen für die Freisetzung der freien Base aus dem Salz darstellen, und in dem das Element (b) zweckmäßig direkten Kontakt mit der Abziehfolie (d) hat, wobei von der Rückschicht (c) und Abziehfolie (d) wenigstens eine vorteilhaft eine Folie aus einem Kunstharz umfaßt, insbesondere eine aluminisierte Folie, und die Kunstharzfolie vorzugsweise aus Polyester besteht.

10. Pflaster entsprechend einem oder mehreren der Ansprüche 1 bis 9, in dem die Größe des Pflasters, falls es zum Kontakt mit der Haut vorgesehen ist, zwischen ca. 10 und ca. 25 cm² liegt.

11. Verfahren zur Herstellung eines Pflasters entsprechend einem oder mehreren der Ansprüche 1 bis 10, umfassend
(i) das Herstellen einer Mischung aus einem physiologisch unbedenklichen Salz eines flüchtigen, pharmazeutisch wirksamen Inhaltsstoffes und einer flüssigen Zusammensetzung aus einem Haftklebermaterial mit einem Lösungs- oder Verdünnungsmittel und Verdampfen des Lösungs- oder Verdünnungsmittels aus dieser Mischung, so daß eine Matrix (a) aus einem homogenen Substrat in Schicht- oder partikulärer Form entsteht,
(ii) Herstellen einer flüssigen Zusammensetzung, enthaltend ein Lösungsmittel oder Verdünnungsmittel und die oben genannte haftklebende Zusammensetzung, die basische Gruppen enthalten kann, welche geeignet sind, die freie Base aus ihrem Salz zu bilden, Aufschichten der Kleberzusammensetzung zu wenigstens einer Schicht, die einen Lochbereich von definierter geometrischer Form aufweisen kann, und Verdampfen dei Löse- oder Verdünnungsmittels aus dieser Schicht, so daß eine Schicht (b) entsteht,
(iii) Laminieren der Matrix (a) mit Schicht (b),
(iv) Auftragen der oben genannten Rückschicht (c) und der oben genannten Abziehfolie (d) auf Matrix (a) und Schicht (b), so, daß entweder die Matrix (a) oder eine für die freie Base durchlässige Schicht von (b) direkten Kontakt mit der Abziehfolie (d) hat,
wobei die mit dem Wirkstoff beladene Matrix (a) und wenigstens eine Schicht (b) eine Klebrigkeit aufweisen, die zur Befestigung des Pflasters auf der Haut ausreicht, und wobei die Matrix (a), bis zur Verarbeitung mit der Zusammensetzung (b), unter solchen Bedingungen gehalten wird, daß das oben genannte Salz nicht zersetzt wird, und wobei die Verfahrensschritte (i) und (ii) in beliebiger Reihenfolge durchgeführt werden.

## Claims

1. A patch for transdermal administration of volatile pharmaceutically active ingredients of chemically basic nature which comprises a multi-element system comprising
(a) a matrix having distributed therein as the drug physiologically acceptable salts of the said volatile active ingredient, the matrix comprising a pressure-sensitive adhesive,
(b) a pressure-sensitive adhesive composition which contains amino groups to liberate the free base from its salt,
(c) a backing layer impermeable to the diffusable ingredients of (a) and (b), and
(d) a release liner impermeable to the diffusable ingredients of (a) and (b),
matrix (a) or at least a part of (b), whichever is in contact with release liner (d), having a tack sufficient for affixing the patch to the skin, any part of (b) positioned between matrix (a) and release liner (d) being permeable to the volatile drug or one of the salts thereof, or both.

2. A patch according to claim 1, wherein the matrix (a) and said parts of (b) are laminated together, and wherein at the time of laminating, the salt is contained only in (a) and the amino groups are contained only in said parts of (b).

3. A patch according to claim 1 or 2, wherein (a) or (b) or both additionally contain the active ingredient in the form of the free base.

4. A patch according to one or more of claims 1 or 3, wherein the salt comprises the hydrochloride or the hydrobromide or both.

5. A patch according to one or more of claims 1 to 4, in which matrix (a) contains a solubilizer for the physiologically acceptable salt, or a permeation enhancer or both.

6. A patch according to one ore more of claims 1 to 5, wherein the active ingredient is deprenyl, which preferably is in the L-form, the deprenyl component suitably being present in an amount of from about 5 to about 75 mg, calculated as deprenyl base.

7. A patch according to one or more of claims 1 to 6, wherein element (b) comprises a plurality of layers and preferably consists of 2 to 3 layers, at least one of which containing basic groups, matrix (a) suitably being laminated between 2 layers of element (b), of which advantageously a layer B₁ is positioned on release liner (d) and a layer B₂ on backing layer (c), layer B₁ having greater tack activity than layer B₂, that layer (b) which is closer to release liner (d) preferably having a lower content of basic groups than that layer (b) which is more remote from release liner (d).

8. A patch according to one or more of claims 1 to 7, wherein matrix (a) comprises a pressure-sensitive adhesive and wherein preferably element (a) is either surrounded to all but one side by element (b), the remaining side being destined for attaching to the skin with the aid of the tack activity of element (a), or surrounded by element (b) such that matrix (a) is in direct contact with backing layer (c).

9. A patch according to one or more of claims 1 to 8, wherein element (b) includes an adhesive polymer containing amino groups constituting the basic groups to liberate the free base from the salt, and wherein element (b) suitably is in direct contact with release liner (d), at least one of the backing layer (c) and the release liner (d) advantageously comprising a foil of a synthetic resin, in particular an aluminized foil, the synthetic resin foil preferably being a polyester.

10. A patch according to one or more of claims 1 to 9, wherein the size of the patch where it is destined to contact the skin, is from about 10 to about 25 cm².

11. A process for preparing a patch according to one or more of claims 1 to 10, which comprises
(i) forming a mixture of a physiologically acceptable salt of a volatile pharmaceutically active ingredient and a fluid composition of a pressure-sensitive adhesive material with a solvent or diluent, and evaporating the solvent or diluent from this mixture to produce a matrix (a) of a homogeneous substrate in layer or particulate form,
(ii) forming a fluid composition containing a solvent or a diluent and said pressure-sensitive adhesive composition which may contain basic groups suitable for the formation of the free base from its salt, stratifying the adhesive composition to at least one layer which may have a hole section of a defined geometrical shape, and evaporating the solvent or diluent from such layer, to form layer (b),
(iii) laminating matrix (a) with layer (b),
(iv) applying said backing layer (c) and said release liner (d) to matrix (a) and layer (b) such that either matrix (a) or a layer of (b) permeable to the free base is in direct contact with release liner (d),
matrix (a), loaded with the active ingredient, and at least one layer (b) having a tack sufficient for affixing the patch to the skin, matrix (a) being maintained, until processing with composition (b), under conditions such that said salt does not suffer decomposition, and process steps (i) and (ii) being carried out in any desired order.

## Revendications

1. Emplâtre pour l'administration percutanée d'ingrédients volatils, chimiquement basiques, pharmaceutiquement actifs sous forme d'un système constitué de plusieurs éléments, comprenant
(a) une matrice dans laquelle sont distribues, à titre de principe actif, des sels physiologiquement acceptables du principe actif volatil, la matrice contenant une colle de contact,
(b) une composition de colle de contact qui contient des groupes amino, pour la libération de la base libre à partir de son sel,
(c) une couche dorsale imperméable aux constituants diffusibles de (a) et de (b) et
(d) un film détachable imperméable pour les constituants diffusibles de (a) et de (b),
la matrice (a) ou du moins une partie de (b), selon que c'est la matrice (a) ou la partie de (b) qui est en contact avec le film détachable (d), présente une adhésivité (tack) qui est suffisante pour la fixation de l'emplâtre sur la peau et chaque partie de (b) disposée entre la matrice (a) et le film détachable (d) étant perméable au principe actif volatil ou à un de ses sels ou aux deux.

2. Emplâtre selon la revendication 1, dans lequel la matrice (a) et les parties de (b) sont stratifiées ensemble et dans lequel, au moment de la stratification, le sel n'est contenu que dans (a) et les groupes amino ne sont contenus que dans les parties de (b).

3. Emplâtre selon la revendication 1 ou 2, dans lequel (a) ou (b) ou les deux contiennent le principe actif en plus sous forme de la base libre.

4. Emplâtre selon une ou plusieurs des revendications 1 à 3, dans lequel le sel comprend l'hydrochlorure ou l'hydrobromure ou les deux.

5. Emplâtre selon une ou plusieurs des revendications 1 à 4, dans lequel la matrice (a) contient un agent de solubilisation pour le sel physiologiquement acceptable ou un agent d'amélioration de la perméation ou les deux.

6. Emplâtre selon une ou plusieurs des revendications 1 à 5, dans lequel le principe actif est le déprényle, de préférence sous forme L, le constituant déprényle étant présent d'une manière appropriée en une quantité d'environ 5 à environ 75 mg, calculé sous forme de base de déprényle.

7. Emplâtre selon une ou plusieurs des revendications 1 à 6, dans lequel l'élément (b) comprend une multitude de couches et est constitué de préférence de 2 à 3 couches, dont au moins une contient des groupes basiques, la matrice (a) étant stratifiée d'une manière appropriée entre deux couches de l'élément (b), dont une couche B₁ est disposée d'une manière avantageuse sur le film détachable (d) et une couche B₂ est disposée sur la couche dorsale (c), la couche B₁ présentant une activité adhésive plus grande que la couche B₂, et la couche (b) qui se trouve à plus grande proximité du film détachable (d) présentant de préférence une teneur plus faible en groupes basiques que la couche (b) qui est plus distante du film détachable (d).

8. Emplâtre selon une ou plusieurs des revendications 1 à 7, dans lequel la matrice (a) contient une colle de contact et dans lequel, de préférence, l'élément (a) est entouré soit de tous les côtés sauf un de l'élément (b), le côté restant étant prévu pour la fixation sur la peau à l'aide de l'activité adhésive de l'élément (a), soit de l'élément (b) de telle manière que la matrice (a) est en contact direct avec la couche dorsale (c).

9. Emplâtre selon une ou plusieurs des revendications 1 à 8, dans lequel l'élément (b) contient un polymère adhésif, qui contient des groupes amino, qui représentent les groupes basiques pour la libération de la base libre à partir du sel, et dans lequel l'élément (b) est d'une manière appropriée en contact direct avec le film détachable (d), au moins un élément parmi la couche dorsale (c) et le film détachable (d) comprenant d'une manière avantageuse un film constitué d'une résine synthétique, en particulier un film pourvu d'une couche d'aluminium, et le film en résine synthétique étant constitué de préférence de polyester.

10. Emplâtre selon une ou plusieurs des revendications 1 à 9, dans lequel la taille de l'emplâtre, si celui-ci est destiné au contact avec la peau, se situe entre environ 10 et environ 25 cm².

11. Procédé pour la production d'un emplâtre selon une ou plusieurs des revendications 1 à 10, comprenant
(i) la préparation d'un mélange à partir d'un sel physiologiquement acceptable d'un constituant volatil, pharmaceutiquement actif et d'une composition liquide constituée d'un matériau de colle de contact avec un solvant ou un diluant et l'évaporation du solvant ou du diluant de ce mélange, de telle manière qu'il se forme une matrice (a) constituée d'un substrat homogène sous forme de couche ou de particules,
(ii) la préparation d'une composition liquide contenant un solvant ou un diluant et la composition adhésive citée ci-dessus, qui peut contenir des groupes basiques, qui sont appropriés pour former la base libre à partir de son sel, l'entassement de la composition de colle en au moins une couche, qui peut présenter une région perforée d'une forme géométrique définie, et l'évaporation du solvant ou du diluant de cette couche, de telle manière qu'il se forme une couche (b),
(iii) la stratification de la matrice (a) avec la couche (b),
(iv) l'application de la couche dorsale (c) citée ci-dessus et du film détachable (d) cité ci-dessus sur la matrice (a) et la couche (b), de telle manière que soit la matrice (a), soit une couche de (b) perméable à la base libre, est en contact direct avec le film détachable (d),
la matrice (a) chargée de principe actif et au moins une couche (b) présentant une adhésivité, qui suffit à la fixation de l'emplâtre sur la peau, et la matrice (a) étant maintenue jusqu'à la transformation avec la composition (b) dans des conditions telles que le sel cité ci-dessus n'est pas décomposé, et les étapes de procédé (i) et (ii) pouvant être réalisées dans un ordre quelconque.
